# EUROPEAN PATENT APPLICATION

(11) **EP 2 229 962 A1**
(43) Date of publication of application: **22.09.2010**
(21) Application number: 09425111.3
(22) Date of filing: 20.03.2009
(51) Int. Cl.: A61L 27/30, A61L 27/54

(54) **Medical-surgical devices with antibacterial coating for human or animal implant and a method for their production**

(71) Applicant: Martelli, Laura, 35126 Padova (IT); Tottolo, Nicola, 35126 Padova (IT); Franco, Maurizio, 31100 Treviso (IT); Martelli, Mario, 35126 Padova (IT)
(72) Inventor: Martelli, Laura, 35126 Padova (IT); Tottolo, Nicola, 35126 Padova (IT); Franco, Maurizio, 31100 Treviso (IT); Martelli, Mario, 35126 Padova (IT)
(74) Representative: Palladino, Saverio Massimo

(57) **Abstract**

The invention relates to medical-surgical devices coated with an antibacterial layer for use in implantology in the human and veterinary field, as well as a method for producing said devices.

## Description

### Field of the invention

The present invention relates to medical-surgical devices coated with an antibacterial layer for use in implantology in the human and veterinary field; the invention also relates to a method for producing said devices.

### Prior art

The use of medical-surgical devices inserted permanently or in any event for lengthy periods into the human or animal body is a widespread practice in various surgical specialties. These devices, hereinafter indicated simply as "implants", are produced from biocompatible materials such as certain metals (in particular titanium), ceramics or hydroxyapatite, and are used in orthopaedic surgery for the reconstruction and replacement of bone or parts thereof, or, in the case of nails, screws, pins and the like, for maintaining bone or parts thereof in the correct mutual position; in dentistry for replacing teeth; in maxillofacial surgery; in cardiovascular surgery, particularly by-passes and stents (cylindrical metal mesh structures introduced into vessels to bring them back to their original diameter following their restriction or obstruction) applied to blood vessels; and in urology for implanting fixed catheters.

The main problem connected with implants is that of bacterial infections. For example, about 4.3% of orthopaedic implants and around 7.4% of cardiovascular implantations realized in the USA give rise to bacterial infections (E. M. Hetrick et al, "Reducing implant-related infections: active release strategies", Chem. Soc. Rev. 2006, 35, 780-789). In such cases, prolonged hospitalization for antibiotic treatment is necessary, with, in the worst cases, a further operation being required to remove and replace the implant.

When the implant is placed into position, a competition is established between the slow integration of the new material into the surrounding tissue and the rapid bacterial adhesion thereto. Should a bacterial infection prevail, the long-term success of the implant is compromised. Once a bacterial colony is established, it produces a biofilm consisting of macromolecules including proteins and polysaccharides which protects the bacterial colony and renders general antibiotic and biocide treatments ineffective (P. Kingshott et al, "Covalent attachment of poly(ethylene glycol) to surfaces, critical for reducing bacterial adhesion", Langmuir, 2003, 19, 6912-6921). It has been observed that the critical period for bacterial colony development is the first few hours after placing the implant into position; consequently, the ability to control bacterial development from the very first hours after implantation is critical.

To confront the problem, two strategies have been proposed.

The first strategy is passive, and consists of modifying the physico-chemical characteristics of the implant material, such that bacterial adhesion thereto is prevented (see for example the aforementioned article by P. Kingshott *et al*.); however, no materials have as yet been found able to completely avoid bacterial adhesion, but at most to slow it down, hence not solving the problem.

The second strategy is active and consists of forming a layer of a material with antibacterial characteristics on the surface of the implant; examples of this approach are reported in the articles "Controlled release of antibiotics from coated orthopedic implants", J. S. Price et al, J. Biomed. Mater. Res., 1996, 30, 281-286; and "Incorporation of different antibiotics into carbonated hydroxyapatite coatings on titanium implants, release and antibiotic efficacy", M. Stigter et al, J. Controlled Release, 2004, 99, 127-137. However, implants coated in this manner present a series of problems: they can only be applied to non-rigid prostheses and in particular those not subjected to friction during their positioning; moreover, antibiotics can lead to the development of bacteria resistant thereto.

As an alternative to antibiotics, silver or salts thereof can be used, their antibacterial properties having long been known. These properties are exploited in the medical field as the metal is active against a wide spectrum of pathogenic agents commonly present in implants (such as P. aeruginosa, E. coli, S. aureus and S. epidermidis) and presents little or no tendency to develop resistance.

The use of silver in various forms in coatings for implants or catheters has been proposed by various research groups.

The article "Silver ion release from antimicrobial polyamide/silver composites", R. Kumar et al, Biomaterials, 2005, 26, 2081-2088, and the aforementioned article by E. M. Hetrick *et al* describe a film coating for implants formed of a polyamide matrix containing Ag⁺ ions; the article "The influence of platinum on the performance of silver-platinum anti-bacterial coatings", A. J. Betts et al, Materials and Design, 2005, 26, 217-222, describes the formation of thin layers of silver/platinum alloys on polyurethane or silicone substrates; finally the article "Antibacterial properties of Ag nanoparticle loaded multilayers and formation of magnetically directed antibacterial microparticles", D. Lee et al, Langmuir, 2005, 21, 9651-9659, describes complex multilayers produced by successive cycles of formation of a polymer substrate, deposition of Ag⁺ ions in this substrate and reduction of the ions to metallic silver. In all the systems described in these articles the silver, in the form of the metal or its ions, is deposited or incorporated in polymer layers; this technology is not suitable for endosseous implants, i.e. orthopaedic devices such as plaques or percutaneous nails, dental implants, etc. because the polymer layer is unable to resist the friction that can develop, upon implantation, between the bone and the surface of the device, and can therefore become detached therefrom.

Other works have concentrated on the use of metallic silver or its alloys which do not present the problem of poor mechanical resistance of the polymers.

The article "Adhesion of bacteria to stainless steel and silver-coated orthopedic external fixation pins", M.A. Wassall et al, J. Biomed. Mater. Res., 1997, 36, 325-330, and the article "Prevention of pin track infection in external fixation with Silver coated pins: clinical and microbiological results", A. Massè et al, J. Biomed. Mater. Res., 2000, 53, 600-604, concern means of orthopaedic fixation (such as nails or screws) formed of steel coated with a layer of metallic silver; the article "Hydrogel/Silver ion-coated urinary catheter reduces nosocomial urinary tract infection rates in intensive care unit patients: a multicenter study", R. A. Bologna et al, Urology, 1999, 54, 982-987, describes urinary catheters coated with metallic silver; finally, the article "Antimicrobial titanium/silver PVD coatings on titanium", A. Ewald, BioMedical Engineering OnLine 2006, 5:22, describes the deposition on titanium of thin layers of a silver/titanium alloy, which exhibit greater mechanical resistance to friction than pure silver.

It is known from the medical literature that silver, to be able to perform an antibacterial action, must be present in the form of Ag⁺ ions at a concentration of the order of at least 1 ppb, equivalent to 1 µg/l (or even 0.1 ppb in the case of certain bacteria); however, these concentrations must not exceed values of about 35 ppb because at higher levels there are cytotoxic effects to the surrounding tissue; see for example the article "Exploring the effects of silver in wound management-What is optimal?", White, R. et al, Wounds, 2006, 18 (no. 11), 307-314, and the US patent 6,267,782 B1.

In the devices of the aforementioned articles, the antibacterial efficacy begins with the oxidation of metallic silver by substances present in biological fluids with which the implants come into contact; oxidation normally has lengthy "induction " periods and so the Ag⁺ ion concentration in the area surrounding the device is in general insufficient to carry out an effective antibacterial activity just in the first few hours after implantation which are crucial, as aforesaid, in preventing the onset of bacterial infection.

Various documents describe methods for increasing Ag⁺ ions emission by deposits of the metal or its compounds.

The aforementioned article by A. J. Betts *et al* describes the formation of an alloy with a more noble metal than silver (in particular platinum) to accelerate its oxidation. In this case however a galvanic pair forms whose thermodynamic properties are controlled only by the Ag/Pt molar ratio and lead to the continuous emission of Ag⁺ ions, independently of the concentration of said ions already present in the surrounding environment; thus even a minimal homeostatic phenomenon, which could control the release of these ions, is lacking and as a consequence their concentration can reach levels such to cause cytotoxic effects, especially in implantation sites where biological fluid quantities, or their exchange rate, are minimal.

US patent 4,476,590 describes deposits of metallic silver or its alloys on prostheses, rendered more reactive by "activation" treatments that are mechanical (for example, by abrasion), thermal (for example, an autoclave treatment at temperatures above 180 °C) or chemical (for example, treatment with 10 to 100 volumes hydrogen peroxide).

US patent 5,958,440 describes the formation of disordered deposits of one or more of a number of metals (including silver), their alloys or their compounds; the term "disordered deposits" refers to deposits consisting of materials in the form of particles less than a micrometer in size, and preferably of the order of a few tens of nanometers, so as to increase the specific surface of the deposit; in this respect, a solid in a finely divided form is known to be more thermodynamically unstable, and hence more reactive, than the same solid in bulk form. To achieve this result, the patent proposes to effect a deposition by the technique known as Physical Vapor Deposition or PVD (also known as sputtering) under specific conditions, including the temperature of the implant on which the deposit is formed, the gas pressure during deposition and the power applied, etc; applying this technique therefore proves to be fairly complex. US patent 6,333,093 B1 by the same author as the previous patent, describes similar deposits consisting of a double layer in which the base layer (in direct contact with the prosthesis) has the previously described disorder characteristics, the top layer being produced from materials and thickness values such as to produce interference phenomena when the prosthesis thus coated is illuminated by visible light.

Finally, US patents 6,113,636 and 6,267,782 B1 describe a wide variety of metals (including silver), their alloys and their compounds, deposited onto prostheses in various manners and combinations, to obtain an effective release of ions with antibacterial action. Examples of possible systems include a combination between a metal and its oxide, obtained by chemical or physical treatments; or a Ag/AgCl combination obtained (see example 2 of US 6,113,636) by deposition of silver chloride onto prostheses of biological origin and subsequent reduction of the surface part of the AgCl layer by light action. In this latter case, in particular, the system is not functional, and can actually prove to be dangerous because the underlying silver chloride has a higher dissolution rate when in contact with bodily fluids than metallic silver, with the result that particles (flakes) of free silver can be produced which can enter the bloodstream and cause damage. Moreover, with this process the antibacterial activity appears after 24 hours of incubation.

None of the known documents describes a system able to release effective quantities of Ag⁺ ions from the very first moments after implant placement and for prolonged periods after said placement, as well as to ensure the maintainance of the concentration of said ions within the range in which they have a bactericidal but non-cytotoxic action.

### Summary of the invention

The object of the present invention is to provide medical-surgical devices for implantation that do not present the problems of the known art, as well as a method for their production.

This object is achieved by means of the present invention which, in a first aspect, relates to a device for implant in the human or animal body, comprising a support produced from a biocompatible material whose surface is at least partially coated with a silver layer, the surface of this being in turn at least partially coated with a compound of silver in which the latter is present in the +1 oxidation state.

### Detailed description of the invention

The inventors have discovered that silver layers, in which at least part of the metal surface is coated with a compound thereof in which silver is present in the +1 oxidation state (also indicated hereinafter as silver(I)), present a combination of characteristics of mechanical strength and release profile of Ag⁺ ions over time, suited to the purposes of bone implantation. In particular, the inventors have verified that the combination of metallic silver and a compound thereof enables a bactericidal but non cytotoxic concentration of Ag⁺ ions to be present from the very first moments after placing the implant in position and for a period of at least a few months, or even longer, this being hard to quantify in advance since it depends on the implantation site and thickness of the deposited silver.

Indeed, in the coated devices of the invention two successive and complementary Ag⁺ ion release phases can be distinguished. In the first phase, which begins immediately after implantation, the (relatively rapid) dissolution of the silver compound which constitutes the outermost layer of the device coating takes place, with release of Ag⁺ ions, such that their concentration is effective even shortly after implantation; this phase also covers the induction period in the oxidation processes of the already exposed metallic silver. Even if the silver layer is initially completely coated with a layer of the compound, dissolution of the latter will generally not be perfectly homogeneous over the entire surface so that, in an intermediate phase, part of the metallic silver surface will be exposed, thus returning the system to the previously described conditions. After said induction period, that can be estimated as a few tens of hours, release of silver ions begins also from the underlying metallic layer whose oxidation in the meantime has started, hence ensuring that an effective concentration is maintained in the long term at the implantation site.

The presence of the two components in the systems of the invention also has a homeostatic effect on Ag⁺ ion concentration; in fact, upon dissolution the silver compound generates the corresponding anion; the concentration of this anion, by means of the solubility product of the compound, has a buffering effect on the quantity of Ag⁺ ions present at the implantation site, whether said ion derives from the compound or from the underlying metallic layer. In particular, such is the case when the implantation site has minimal volumes of biological fluids, their exchange rate also being minimal.

The support on which the silver layer is present can be produced from any known biocompatible material used for implant production, such as ceramics, metals or alloys; in particular, materials suitable for the production of the support are steel and especially titanium or its alloys. The shape of the support is that of the final device and can for example be a prosthesis, a screw, etc.

The surface of the support is coated with a layer of metallic silver, which can be formed by the methods described below. It is not necessary to coat all the surface of the support with silver: for example, in the case of pins for dental prostheses, it may be sufficient to silver coat only that part of the implant which, once installed, is positioned at the level of the bone neck, i.e. the region in which the bacteria enter towards the implant surface.

The thickness of the silver layer can vary within broad limits. The minimum thickness is in the order of 10 nm, to ensure the presence of a sufficient metal quantity to provide antibacterial activity for a sufficiently long time period. This thickness can be of the order of some millimetres in size, especially if depressions in the surface of the support are filled with the metal. The thickness need not be uniform over the entire layer, and indeed in some cases it may be preferable to have silver layers with an undulated or corrugated surface in order to limit, in the depressions thereof, the extent of scraping-off of the overlying silver compound during the medical-surgical device installation procedure. The layer can also be discontinuous, i.e., composed of silver "islands" separated by areas where the surface of the support is exposed.

The free surface of the metallic silver layer is itself at least partially coated by a compound of silver in which the latter is present in the +1 oxidation state. The compound can be for example a salt or a complex. Said compound must not have too high a solubility in the fluids surrounding the implant to avoid that the Ag⁺ ion concentration becomes cytotoxic; this limited solubility must however be accompanied by rapid solubilisation kinetics in order to provide antibacterial activity in the shortest possible time. By knowing at least broadly the concentrations of the most common anions in organic fluids, and by also knowing the solubility products of silver salts with these anions, it is possible to ascertain which of these salts will give rise to a non-cytotoxic concentration; for example, acetate or nitrate are unusable because they result in excessive Ag⁺ concentrations. This control of Ag⁺ concentration would be difficult to achieve with other known systems, such as resins loaded with said ions or galvanic pairs (for example the Pt/Ag pair, as previously discussed). Salts suitable for the purposes of the invention are arsenate (Ag₃AsO₄), carbonate (Ag₂CO₃), phosphate (Ag₃PO₄), sulfate (Ag₂SO₄), bromide (AgBr), chloride (AgCl), iodide (Agl), sulfite (AgSO₃), thiocyanate (AgSCN) and silver(I) complexes such as silver sulfadiazine (empirical formula AgC₁₀H₉N₄O₂S; CAS No. 22199-08-2).

The extent to which the metallic silver is coated with the compound thereof can vary within wide limits, for example from about 20% to 100% of the surface of the metal. The thickness of the compound layer can be between about 10 nm and 10µm. The function of the compound is to ensure the presence of an effective Ag⁺ ion concentration from just after implant installation (by virtue of its ion release rate being greater than that of the metal) until it combines with the Ag⁺ deriving from the oxidized metallic silver when the metallic silver oxidation process becomes efficient; accordingly, less coverage will require a greater thickness and vice-versa, within the aforesaid limits.

In a second aspect, the invention relates to a method for the production of the aforedescribed medical-surgical devices.

The method of the invention consists in:
- depositing a metallic silver layer on at least part of the surface of a support produced from a biocompatible material and having the shape of the final device; and
- subjecting the silver layer thus obtained to chemical, electrochemical or physical treatments in order to coat at least part of the surface of said layer with a silver compound in which silver is present in the +1 oxidation state.
   The first operation of the method is to deposit the metallic silver layer onto the support. The known methods for implementing this operation are many, and comprise chemical methods such as the known process of silver salt (typically AgNO₃) reduction with reducing sugars in a solution into which the support has been immersed; the technique of deposition from chemical vapors, known as Chemical Vapor Deposition or its acronym CVD (and variants thereof); or galvanic deposition. As an alternative, physical methods can be used such as evaporation of the metal under vacuum and condensation thereof onto the support in suitable chambers; the technique of Physical Vapor Deposition, also known as sputtering; Plasma Spray Deposition; and various other techniques.

In these techniques, the desired thickness of the silver layer can be achieved by controlling the quantity of reagents (for example in the case of reduction with sugars) or deposition time, such as in CVD, in galvanic deposition and in physical techniques in general. Should the silver layer not be required to coat the entire support, the area of the deposit can be defined by suitable masking, or by only partially immersing the support in reagent solutions, or as a direct consequence of directional deposition techniques such as sputtering. In any event, by rotating the support during deposition, a complete coating can be achieved even with directional techniques. Finally, the morphology of the silver layer can be controlled as well, within certain limits, by the technique selected: for example, the deposits obtained by Plasma Spray Deposition in the initial phases of formation consist of "islands" as a result of the impact and solidification of liquid metal droplets onto the support.

In the second operation of the method the external surface of the silver layer, or a part thereof, is coated with a silver compound as previously stated. This coating operation can consist of actually depositing the compound, or of reacting the surface part of the silver layer to obtain the desired compound.

In the first case (depositing the compound) techniques that can be used are the aforementioned of Plasma Spray Deposition or reactive sputtering, in which silver deposition (in the form of ion clusters) is made to happen in an atmosphere of, for example, chlorine to obtain silver chloride.

In the second case (reacting the surface part of the previously formed silver layer) use can be made of metal anodization in solutions containing the anion corresponding to the desired compound, or chemical oxidation, or ionic implantation followed by annealing to bring the obtained system to chemical equilibrium. The areas of deposition, thickness and morphology of the silver compound can be controlled in a similar manner to that described for the silver layer.

The invention will be further described by the following examples.

### EXAMPLE 1

This example relates to metallic silver deposition on supports representative of those actually used in implantology.

A series of titanium bars of technical grade 4 and 1 cm x 2 cm x 10 cm in size are prepared. The bars are cleaned a first time with ultrasound treatment in double-distilled water at 40 °C for 15 minutes, then washed with acetone and finally subjected to a second ultrasound treatment exactly as before. The bars prepared in this manner are stored in a desiccator (over P₂O₅) until required.

The bars are then coated with metallic silver in an Edwards E3064 evaporation chamber, with heating by electron bombardment of the crucible of the metal and control of the deposited thickness by a quartz microbalance. The deposition rate is between 0.1 and 0.2 nanometers per second (nm/s) and the deposited thickness is 50 nm.

### EXAMPLE 2

This example relates to the formation of a silver compound by electrochemical means.

Some of the bars produced as described in example 1 are immersed in an electrochemical bath and connected to its anode. The solution in the bath consists of 0.1 M NaCl and 0.1 M NaClO₄ (the latter having the role of supporting electrolyte). The operational voltage is set to between 0.0 and 0.2 V against Ag/AgCl, obtaining a current of about 15 mA/cm². To control the AgCl layer thickness during growth, a coulombometric measurement of the anode charge is used and found to be about 10 µC per centimetre square per nanometer of thickness.

The bars coated in this manner are washed with distilled water and stored in the dark and under inert atmosphere.

### EXAMPLE 3

This example relates to the formation of a silver compound by chemical means.

Some of the bars produced as described in example 1 are immersed in a solution of 0.05 M FeCl₃ for one minute at ambient temperature. A coating consisting of AgCl of thickness of about 100 nm is obtained on the silver. The bars thus coated are washed with distilled water and stored in the dark and under inert atmosphere.

### EXAMPLE 4

This example relates to a test for evaluating antibacterial activity of the bars of the invention and bars of the known art.

The antibacterial activity of bars produced according to examples 2 and 3 is measured by inserting them into a bacterial suspension consisting of the Gram-negative E. coli pTAQ strain in distilled water at a concentration of 1.1 ± 0.2 x 10⁶ colony forming units per millilitre (cfu/ml). The results, expressed in percentage of bacterial survival at different times, are given in table 1. As a comparison analogous survival results obtained with bars of titanium alone and with titanium bars coated with silver only (produced in example 1) are given.

**Table 1**

| Incubation time (hours) | Survival with titanium bars (%) | Survival with titanium + silver bars (%) | Survival with titanium + silver + AgCl bars (%) |
|---|---|---|---|
| 0 | 100 | 100 | 100 |
| 3 | 100 | 90 ± 10 | 0 |
| 24 | 100 | 25 ± 3 | 0 |
| 48 | 100 | 0 | 0 |

As can be seen from the results in table 1, titanium alone has no bactericidal capacity, so implants produced from this metal alone are exposed to bacterial colonization; the titanium bars coated with silver alone only develop an antibacterial capability over time and reach full efficiency only after 24 hours when the bacterial infection can already be established; the bars of the invention instead develop full antibacterial activity (0% survival) right from the first check (3 hours after implantation), this capability being maintained for the entire observation period. Similar results to the aforegiven have been obtained with bars coated with Ag/AgCl obtained by reacting silver coated bars in a chlorine atmosphere, or in oxidizing solutions containing chlorine following dismutation of suitable reagents (e.g. sodium hypochlorite); likewise, similar results have been obtained by forming silver bromide or silver iodide layers over the metallic silver layer using the electrochemical method of example 2, but substituting the sodium chloride with the sodium salt of the corresponding anion. These results were not obtainable with the systems of the known art, in which antibacterial activity was apparent about 24 hours or more after implantation.

## Claims

1. Medical-surgical device for implant in the human or animal body, comprising a support produced from a biocompatible material whose surface is at least partially coated with a metallic silver layer, the surface of this being in turn at least partially coated with a compound of silver in which the latter is present in the +1 oxidation state.

2. Device according to claim 1 wherein said support is produced from a biocompatible metal or alloy.

3. Device according to claim 2 wherein said support is produced from titanium or an alloy thereof.

4. Device according to claim 1 wherein said metallic silver layer has a minimum thickness of 10 nm, which is not necessarily uniform throughout the layer.

5. Device according to claim 1 wherein said metallic silver layer is discontinuous.

6. Device according to claim 1 wherein said silver compound is chosen from arsenate (Ag₃AsO₄), carbonate (Ag₂CO₃), phosphate (Ag₃PO₄), sulfate (Ag₂SO₄), bromide (AgBr), chloride (AgCl), iodide (Agl), sulfite (AgSO₃), thiocyanate (AgSCN) and silver(I) complexes.

7. Device according to claim 6 wherein silver sulfadiazine (AgC₁₀H₉N₄O₂S) is used as the silver(I) complex.

8. Device according to claim 1 wherein said silver compound is the chloride (AgCl).

9. Device according to claim 1 wherein the extent to which the metallic silver layer is coated with said compound is comprised between 20% and 100% of the metal surface.

10. Method for producing the medical-surgical device of claim 1, consisting in:
- depositing a metallic silver layer on at least part of the surface of a support produced from a biocompatible material and having the shape of the final device; and
- subjecting the silver layer thus obtained to chemical, electrochemical or physical treatments in order to coat at least part of the surface of said layer with a compound of silver in which the latter is present in the +1 oxidation state.

11. Method according to claim 10 wherein the technique for depositing said metallic silver layer is chosen from: reduction of silver salts with reducing sugars; deposition from chemical vapors; galvanic deposition; evaporation of the metal under vacuum and condensation thereof onto the support; Physical Vapor Deposition; and Plasma Spray Deposition.

12. Method according to claim 11 wherein when the technique for depositing said metallic silver layer is reduction of silver salts with reducing sugars, the silver layer thickness is controlled by regulating the quantity of reagents.

13. Method according to claim 11 wherein the technique for depositing said metallic silver layer is one among deposition from chemical vapors, galvanic deposition, evaporation of the metal under vacuum and condensation thereof onto the support, Physical Vapor Deposition and Plasma Spray Deposition, and the silver layer thickness is controlled by controlling the deposition time.

14. Method according to claim 10 wherein the treatment for coating at least part of the surface of the metallic silver layer with a compound thereof is chosen from Physical Vapor Deposition in the presence of a reactive gas, Plasma Spray Deposition, metal anodizing in solutions containing the anion of the desired compound, chemical oxidation and ionic implantation followed by annealing.
